# EUROPEAN PATENT SPECIFICATION

(11) **EP 3 246 311 B1**
(45) Date of publication and mention of the grant of the patent: **24.06.2020**
(21) Application number: 17172060.0
(22) Date of filing: 19.05.2017
(51) Int. Cl.: C07C 403/24

(54) **PROCESS FOR THE PRODUCTION OF LUTEIN**
VERFAHREN ZUR HERSTELLUNG VON LUTEIN
PROCÉDÉ DE PRÉPARATION DE LUTEINE

(30) Priority: 21.05.2016 IN 201641017567
(43) Date of publication of application: 22.11.2017
(73) Proprietor: National Institute of Ocean Technology, 600100 Chennai, Tamil Nadu (IN)
(72) Inventor: JOSEPH, Leema Thilakam Mary, 600100 Tamil Nadu (IN); DHASSIAH, Peter Magesh, 600100 Tamil Nadu (IN); THALAVAI SHIVASANKARASUBBIAH, Kumar, 600100 Tamil Nadu (IN); KEPPAYAN, Thirupathi, 600100 Tamil Nadu (IN); GOPAL, Dharani, 600100 Tamil Nadu (IN); RAMALINGAM, Kirubagaran, 600100 Tamil Nadu (IN); MALAYATH ARAVINDAKSHAN, Atmanand, 600100 Tamil Nadu (IN)
(74) Representative: HGF Limited

(56) References cited:
- WO-A1-2013/032412
- WO-A1-2014/023917

## Description

### FIELD

The present disclosure relates to a process for the production of lutein from microalgal biomass.

### BACKGROUND

Lutein, a naturally occurring xanthophyll carotenoid, is a free radical scavenging antioxidant and is essential for protecting the cellular components of vital organs from oxidative damage. Lutein is a vital macular pigment in the retina of the eye and protects the eye from the ionizing effect of blue light. Lutein is basically of plant origin and is available in very low quantities in a number of green leaves and vegetables. Hence, commercial scale production of lutein from these sources is not economically feasible. Commercially available lutein is synthetic and the major natural source for commercial production of lutein is petals of marigold flowers. The concentration of lutein in marigold varies from 5% to 50% and occurs mostly in the di-ester form and the free lutein content can be as low as 0.03%. Production of lutein from marigold petals is labor intensive, demands a large area of agricultural land and copious fresh water, and competes with food crops. Further, marigiold has long crop duration and low lutein content. WO 2013/032412 discloses methods of extraction of highly purified lutein/xanthophylls obtained from natural materials. The method according to this invention includes the modification of natural lutein ester in the natural material to free lutein and/or low molecular weight lutein ester. The lutein so formed can be extracted with supercritical fluids as crude lutein. The crude lutein can be further purified with chromatography in order to obtain the highly purified lutein. Therefore the above mentioned method involves a step of pre-treatment by hydrolysis with low molecular weight acid or transesterification before supercritical fluid extraction, thereby making the process tedious.

Some of the known methods suggest analysis, estimation and extraction of lutein from freshwater microalgae. But, freshwater is scarce in many of the tropical countries. Hence a process for producing lutein from marine microalgae is the need of the hour for commercial production of lutein.

Further, in the known processes, harvesting of microalgae is carried out by energy intensive centrifugation and extraction of lutein is done using harsh solvents. Processes like centrifugation, sedimentation, filtration under vacuum, are also used. These processes are energy intensive and contribute to a hike in the production cost. There is, therefore, felt a need for an efficient and economical process for the production of lutein.

### OBJECTS

Some of the objects of the present disclosure, which at least one embodiment herein satisfies, are as follows:
It is an object of the present disclosure to ameliorate one or more problems of the prior art or to at least provide a useful alternative.

An object of the present disclosure is to produce lutein from marine microalgal biomass.

Another object of the present disclosure is to provide an efficient process for the production of lutein from marine microalgal biomass.

Yet another object of the present disclosure is to provide an economical process for the production of lutein from marine microalgal biomass.

Still another object of the present disclosure is to provide a process that employs minimal amount of permissible organic solvents for the production of lutein from marine microalgal biomass.

Other objects and advantages of the present disclosure will be more apparent from the following description, which is not intended to limit the scope of the present disclosure.

### SUMMARY

The present disclosure provides a process for the production of lutein from harvested microalgal biomass. The process of the present disclosure comprises the following steps:
i. Subjecting dried harvested microalgal biomass having particle size in the range of 75 microns to 100 microns to one of supercritical fluid extraction process or ultrasonication extraction process to obtain a mixture comprising lutein and at least one fluid medium;
ii. saponifying the mixture, using at least one alkali, at a temperature ranging from 55 °C to 65 °C and for a time period ranging from 2 hrs to 2.5 hrs to produce a saponified slurry, wherein the ratio of said alkali to said fluid medium is 1:9;
iii. centrifuging the saponified slurry at a speed ranging from 2500 rpm to 3000 rpm to separate a wet mass comprising lutein; and
iv. concentrating the wet mass at a temperature in the range of 25 °C to 30 °C to obtain lutein.

The process of extracting lutein from harvested microalgal biomass using supercritical fluid extraction process comprises the following steps:
- introducing the dried harvested microalgal biomass having particle size in the range of 75 microns to 100 microns in an air tight vessel;
- passing supercritical carbon dioxide (CO₂) in the air tight vessel;
- adding at least one fluid medium in the air tight vessel, wherein the ratio of the amount of the microalgal biomass to the amount of at least one fluid medium is in the range of 1:0.3 to 1:0.6;
- increasing the pressure of the air tight vessel to a pressure in the range of 350 bar to 380 bar, and temperature of the air tight vessel to a temperature in the range 60 °C to 65 °C for a time period ranging from 0.5 hrs to 4.0 hrs;
- stirring the contents of the air tight vessel to obtain a slurry stream containing lutein; and
- reducing the pressure of the slurry stream to a pressure in the range of 10 bar to 20 bar, to obtain the mixture containing lutein and at least one fluid medium.

The ultrasonication extraction process to extract lutein from the microalgal biomass comprises the following steps:
a. introducing the dried harvested microalgal biomass having particle size in the range of 75 microns to 100 microns, in a vessel;
b. adding at least one fluid medium in the vessel containing the dried harvested microalgal biomass, wherein the ratio of the dried harvested microalgal biomass to the at least one fluid medium is in the range from 1:10 to 1:40, to obtain a mixture comprising the microalgal biomass and at least one fluid medium;
c. stirring the mixture for a time period ranging from 2 minutes to 10 minutes to obtain a slurry; and
a. ultrasonicating the slurry, at an acoustic intensity of 167 W/cm² and an ultrasonic frequency of 20 kHz, for a time period ranging from 15 minutes to 60 minutes in a chilled bath to obtain the mixture containing lutein and at least one fluid medium.

The lutein thus obtained can be further treated with a salt solution comprising at least one salt selected from the group consisting of NaCl and KCl, to obtain lutein of 90% purity.

The microalgal biomass of the present disclosure can be at least one microalgae selected from the group consisting of *Chlorella vulgaris, Chlorella sorokiniana, Chlorella pyrenoidosa, Neochloris aquatica, Dunaliella salina* and *Tetraselmis gracilis.*

The alkali used in the saponification process can be at least one selected from the group consisting of KOH, NaOH. The at least one fluid medium used in the process of the present disclosure can be selected from the group consisting of ethanol, isopropanol and a mixture containing ethanol and tetrahydrofuran in the ratio of 2:1 The concentration of at least one fluid medium used in the process of the present disclosure can be in the range of 98% to 99.8%.

### DETAILED DESCRIPTION

In one aspect of the present disclosure, there is provided a process for the production of lutein from marine microalgal biomass.

The microalgal biomass used in the process of the present disclosure can be harvested by any system and process, e.g. the system of harvesting microalgae disclosed by the present inventors in the co-pending application 1991/CHE/2015.

In accordance with the process of the present disclosure, the harvested microalgal biomass can be first subjected to one of supercritical fluid extraction process and ultrasonication extraction process to obtain a mixture comprising lutein and at least one fluid medium.

The supercritical fluid extraction process of the present disclosure uses CO₂, which after the extraction process can be recycled back to the process, thereby making the process of the present disclosure economical.

Further, the ultrasonication extraction process of the present disclosure uses minimal amount of organic solvents as fluid medium, to extract lutein from the microalgal biomass, which results in an environment friendly process.

In accordance with one exemplary embodiment of the present disclosure, ethanol is used as the fluid medium.

The mixture comprising lutein and at least one fluid medium is then saponified using at least one alkali. Saponification can be carried out at a temperature in the range of 55 °C to 65 °C and for a time period in the range of 2 hrs to 2.5 hrs, to produce a saponified slurry.

In accordance with one exemplary embodiment of the present disclosure, potassium hydroxide (KOH) is used as an alkali.

The saponified slurry thus obtained, is centrifuged at a speed ranging from 2500 rpm to 3000 rpm, to separate a wet mass. The wet mass obtained after centrifuging the saponified slurry, comprises lutein and at least one fluid medium.

In accordance with the present disclosure, the wet mass is concentrated at a temperature in the range of 25 °C to 30 °C in an evaporator to reduce the fluid medium present in the wet mass and to obtain lutein.

In accordance with the present disclosure, lutein thus obtained can be further treated with a salt solution comprising at least one salt selected from the group consisting of NaCl and KCl, to obtain lutein of 90% purity.

In accordance with the present disclosure, the supercritical fluid extraction process comprises the following steps:
Dried harvested microalgal biomass, having particle size in the range of 75 microns to 100 microns, is loaded in an air tight vessel and then the supercritical CO₂ is passed in the air tight vessel. A fluid medium is also added to the air tight vessel containing the microalgal biomass and the supercritical CO₂. The ratio of the amount of the microalgal biomass to the amount of at least one fluid medium added to the air tight vessel is in the range of 1:0.3 to 1:0.6.

After that, the pressure of the vessel containing harvested microalgal biomass, the supercritical CO₂, and the fluid medium, is increased to a pressure in the range of 350 bar to 380 bar, and the temperature is increased to a temperature in the range of 60 °C to 65 °C for a time period in the range of 0.5 hrs to 4 hrs.

In accordance with the present disclosure, the contents of the vessel are stirred to obtain a slurry stream containing lutein. The supercritical CO₂ along with the fluid medium diffuses into the microalgal biomass and dissolves the lutein to produce a slurry stream containing supercritical CO₂ with dissolved lutein and at least one fluid medium.

The slurry stream containing the dissolved lutein is separated and the pressure of the slurry stream is reduced to a pressure in the range of 10 bar to 20 bar to obtain the mixture comprising lutein and at least one fluid medium.

In accordance with the present disclosure, the ultrasonication extraction process comprises the following steps:
Dried microalgal biomass, having particle size in the range of 75 microns to 100 microns is loaded in a vessel. A fluid medium is also added to the vessel containing the dried microalgal biomass to obtain a mixture comprising microalgal biomass and at least one fluid medium.

In accordance with the present disclosure, the ratio of the microalgal biomass to at least one fluid medium added to the vessel, is in the range of 1:10 to 1: 40.

The mixture comprising the microalgal biomass and at least one fluid medium, is then stirred for a time period in the range of 2 minutes to 10 minutes to obtain a slurry comprising the microalgal biomass and at least one fluid medium. The slurry, thus obtained, is ultrasonified to obtain a mixture containing lutein and at least one fluid medium.

In accordance with the present disclosure, the stirred mixture is exposed to ultrasound, at an acoustic intensity of 167 W/cm² and an ultrasonic frequency of 20 kHz, for a time period ranging from 15 minutes to 60 minutes in a chilled bath.

In accordance with the present disclosure, the chilled bath is maintained at a temperature in the range of 5 °C to 30 °C, typically the temperature of the chilled bath can be 25 °C.

In accordance with the present disclosure, the microalgal biomass can comprise at least one microalgae selected from the group consisting of *Chlorella vulgaris, Chlorella sorokiniana, Chlorella pyrenoidosa, Neochloris aquatica, Dunaliella salina* and *Tetraselmis gracilis.*

In accordance with the present disclosure, the alkali is at least one selected from the group consisting of KOH and NaOH. The ratio of said alkali to said at least one fluid medium is typically 1:9.

In accordance with the present disclosure, the at least one fluid medium is selected from the group consisting of ethanol, isopropanol, and a mixture containing ethanol and tetrahydrofuran in the ratio of 2:1. The concentration of the fluid medium is in the range of 98% to 99.8%.

The present disclosure is further described in light of the following experiments which are set forth for illustration purpose only and not to be construed for limiting the scope of the disclosure. The following experiments can be scaled up to industrial/commercial scale and the results obtained can be extrapolated to industrial scale.

### Experiment 1: Extraction of lutein using Supercritical CO₂:

100 gm of harvested freeze dried microalgal biomass containing *Chlorella vulgaris and Chlorella sorokiniana* having particle size of 100 microns, was loaded in an air tight extraction vessel. Liquid CO₂ was first heated to a temperature of 62 °C, to attain supercritical condition and then passed into the extraction vessel at a flow rate of 20 g/min. In addition to the super critical CO₂, 0.5 ml of ethanol having a concentration of 99.8%, was also added into the extraction vessel, at a flow rate of 0.5 ml/min. The ratio of the microbial biomass to the ethanol, loaded in the extraction vessel, was 1:0.3. Then, the pressure of the extraction vessel was increased to 350 bar and the temperature of the extraction vessel was maintained at 62 °C for an hour. The contents of the extraction vessel were stirred to obtain a slurry stream. The supercritical CO₂, along with ethanol, diffused into the microalgal biomass and formed a slurry stream containing supercritical CO₂, ethanol and dissolved lutein. The slurry stream containing dissolved lutein was removed and the lutein dissolved in the supercritical CO₂ was separated by reducing the pressure of the slurry stream to 20 bar. The separated CO₂ was cooled and recycled. The extracted lutein was saponified to make it free of neutral lipids. The extracted lutein was saponified with 10 M KOH containing 2.5% ascorbic acid, in an amount of 1 ml for every 9 ml of ethanol, at a temperature of 60 °C for a time period of 2 hrs. The saponified slurry, thus obtained, was centrifuged at a speed of 3000 rpm for a time period of 10 minutes, and a wet mass containing extracted lutein along with ethanol was obtained. The wet mass was concentrated in a rotary evaporator at a temperature of 28 °C to evaporate ethanol. The lutein, thus produced, after evaporating ethanol, was of 64 % purity.

Further, the lutein of 64% purity, was purified with 10% NaCl solution. 20 ml of 10% NaCl solution was added for every 10 ml of extract to obtain a mixture containing lutein in 10% NaCl solution. The mixture was stirred at a speed of 150 rpm for 15 minutes to obtain a stirred mixture. The stirred mixture was transferred into a separating funnel and retained in the dark for 15 minutes to separate the stirred mixture into two phases viz. an organic phase containing lutein and an aqueous phase. The aqueous phase was removed from the funnel and discarded. The organic phase containing lutein, was washed twice with 2 ml of 10% NaCl solution followed by evaporation in a rotary evaporator to remove the water present in the organic phase. The organic phase thus obtained after evaporation, was stored at a temperature of minus 20 °C. The lutein thus obtained from the organic phase had a purity of 90%.

The effects of operating parameters on the supercritical fluid extraction process of lutein were investigated. Analyses of the extract was done by high performance liquid chromatography (HPLC) equipped with UV-Vis detector and autosampler. A reference extraction with a fluid medium containing methanol:dichloromethane in a ratio of 3:1, was used as a comparison to evaluate the performance of the supercritical extraction process. The effect of temperature and pressure on the lutein extraction (mg/g of dried biomass) was studied at a CO₂ flow rate of 20 g/min. The results obtained are tabulated in Table 1 herein below.

**Table 1: Temperature and pressure effect on the extraction of lutein using Supercritical fluid Extraction (SFE) process**

| **1. Pressure = 350 Bar** | | | | |
|---|---|---|---|---|
| **Temperature** | 72 °C | 62 °C | 52 °C | 42 °C |
| | 7.45132 | 7.664214 | 7.102819 | 4.859721 |
| | 7.821732 | 7.593779 | 5.78562 | 5.625649 |
| | 6.95265 | 8.64375 | 5.855444 | 5.625649 |
| **Lutein (mg/g)** | 6.576767 | 8.025221 | 7.103143 | 5.233231 |
| | 7.495871 | 7.516305 | 5.870366 | 4.991195 |
| | 7.362398 | 9.518425 | 6.457403 | 4.520417 |
| **Mean (mg/g)** | 7.27679 | 8.160282 | 6.362466 | 5.142644 |
| **Standard deviation (SD)** | 0.442232 | 0.784634 | 0.622531 | 0.439305 |

| **2. Pressure** = **250 bar** | | | | |
|---|---|---|---|---|
| **Temperature** | 72 °C | 62 °C | 52 °C | 42 °C |
| **Lutein (mg/g)** | 4.861494 | 5.176448 | 3.214155 | 1.8119 |
| | 5.049183 | 5.411694 | 4.518552 | 1.6443 |
| | 4.690456 | 5.6405 | 4.56115 | 1.808733 |
| | 4.656179 | 5.2949 | 4.109503 | 1.9896 |
| | 4.976603 | 5.3589 | 3.698553 | 1.5627 |
| | 4.6 | 5.5438 | 3.328698 | 1.4948 |
| **Mean (mg/g)** | 4.805653 | 5.404374 | 3.905929 | 1.718672 |
| **SD** | 0.184149 | 0.168078 | 0.584354 | 0.184489 |

| **3. Pressure = 150 bar** | | | | |
|---|---|---|---|---|
| **Temperature** | 72 °C | 62 °C | 52 °C | 42 °C |
| **Lutein (mg/g)** | 1.3572 | 2.675062 | 1.69233 | 1.24 |
| | 1.61251 | 2.691478 | 1.200357 | 0.883752 |
| | 1.721411 | 3.394416 | 1.281424 | 0.943436 |
| | 1.820391 | 2.886275 | 1.355104 | 0.997683 |
| | 1.61 | 3.044074 | 1.409426 | 1.037677 |
| | 2.260733 | 2.886275 | 1.978141 | 1.102107 |
| **Mean (mg/g)** | 1.730374 | 2.929597 | 1.48613 | 1.034109 |
| **SD** | 0.302359 | 0.266234 | 0.293672 | 0.125844 |

It was observed that the quantity of lutein extracted increased with increase in temperature from 1.03 ± 0.12 mg/g at 150 bar and 42 °C to 8.16 ± 0.78 mg/g at 350 bar and 62 °C, but declined thereafter at 72 °C to 7.27 ± 0.44 mg/g. The results thus obtained showed that it is optimal to work at a pressure of 350 bar and at a temperature of 62 °C to extract lutein from the microalgae biomass.

### Experiment 2: Extraction of lutein using ultrasonication extraction process:

4 g of dried harvested microalgal biomass containing *Chlorella vulgaris* and *Chlorella sorokiniana,* having particle size of 100 microns, was added into a 500 ml glass beaker. 80 ml of 99.8% ethanol was added to the beaker containing the dried biomass and the resultant mixture was stirred for a time period of 2 minutes to obtain a slurry. After that, the slurry was ultrasonified for 60 minutes, by dipping ultrasound probe tip into the slurry kept in a chilled bath. The ultrasonication process used for generating ultrasound, was operated at an ultrasonic frequency of 20 kHz and an acoustic intensity of 167W/cm². The ultrasound, at this frequency, produce cavitation bubbles, which implode and cause the cell wall of the microalgae to rupture. The ruptured cell wall, allow ethanol to penetrate into the cell and increase mass transfer along with good mixing. The lutein present in the cell wall, dissolved in ethanol and a mixture containing lutein dissolved in ethanol was obtained.

The mixture containing lutein dissolved in ethanol, was saponified with 10 M KOH containing 2.5% ascorbic acid, in an amount of 1 ml for every 9 ml of ethanol, at a temperature of 60 °C for a time period of 2 hrs. The saponified slurry, thus obtained, was centrifuged at a speed of 3000 rpm for a time period of 10 minutes, to produce a wet mass containing lutein along with ethanol. The wet mass was concentrated in a rotary evaporator at a temperature of 28 °C to evaporate ethanol followed by filtering using 0.22 µm PTFE filters to obtain lutein. The lutein thus obtained had a purity of 64%.

Further, the lutein of 64% purity, was purified with 10% NaCl. 20 ml of 10% NaCl solution was added for every 10 ml of extract to obtain a mixture containing lutein in 10% NaCl solution. The mixture was stirred at a speed of 150 rpm for 15 minutes to obtain a stirred mixture. The stirred mixture was transferred into a separating funnel and retained in the dark for 15 minutes to separate the stirred mixture into two phases viz. organic phase containing lutein and an aqueous phase. The aqueous phase was removed from the funnel and discarded. The organic phase containing lutein, was washed twice with 2 ml of 10% NaCl solution followed by evaporation in a rotary evaporator to remove the water present in the organic phase. The organic phase thus obtained after evaporation, was stored at a temperature of minus 20 °C. The lutein thus obtained from the organic phase had a purity of 90%.

The change in lutein yield from micro algal biomass at different extraction times was investigated. The data thus obtained is shown in Table 2.

**Table 2: Effect of the duration of extraction on lutein yield**

| **S.No** | **Ultrasonication time** | **Lutein content (mg/g)** |
|---|---|---|
| 1. | 5 minutes | 5.44 ± 0.42 |
| 2. | 15 minutes | 6.48 ± 0.56 |
| 3. | 30 minutes | 6.95 ± 0.45 |
| 4. | 45 minutes | 7.57 ± 0.57 |
| 5. | 60 minutes | 8.76 ± 0.76 |
| 6. | 90 minutes | 7.65 ± 0.67 |
| 7. | 120 minutes | 6.88 ± 0.43 |

It is evident from the Table 2, that the extraction yield increases exponentially till a few minutes (5 minutes), later increases gradually (15 minutes) and then decreases (after 60 minutes). This was because the bubbles formed due to cavitation collapsed and released a large amount of energy to the surrounding which causes disruption of cell wall. The initial sharp increase in the rate of extraction was due to large lutein concentration gradient between the extracting fluid medium and the cell, and also due to easier extraction of constituents from the outer part. With increasing extraction time, the mass transfer also increases. But later, the extraction becomes difficult due to the presence of lutein in the interior location within the cells. Further, as the release of lutein from the cell increases with time, the fluid medium becomes saturated with lutein. So, after that, there was lesser mass transfer and extraction of lutein.

**Effect of biomass to fluid medium ratio:** The extraction yield of lutein was measured at different microalgal biomass to fluid medium ratios ranging from 1:10 to 1:50 and the results are tabulated in Table 3.

**Table 3: Effect of microalgal biomass to fluid medium ratio on extraction yield of lutein.**

| **S.No** | **Solid: fluid medium ratio** | **Lutein content (mg/g)** |
|---|---|---|
| 1. | 1:10 | 4.02 ± 0.21 |
| 2. | 1:20 | 7.01 ± 0.33 |
| 3. | 1:30 | 7.49 ± 0.30 |
| 4. | 1:40 | 8.26 ± 0.27 |
| 5. | 1:50 | 7.65 ± 0.47 |

It is evident from the Table 3 that the extraction yield of lutein increased as the microalgal biomass to fluid medium ratio was increased from 1:10 to 1:40, and the microalgal biomass to fluid medium ratio of 1:40 gave the maximum yield of lutein. With further increase in the microalgal biomass to fluid medium ratio, the extraction yield of lutein decreased. This is because as the biomass concentration increases, the fluid medium slowly becomes saturated, resulting in poor interaction of the fluid medium with the biomass, which reduces the extraction efficiency.

### TECHNICAL ADVANCEMENTS

The present disclosure described herein above has several technical advantages including, but not limited to, the realization of:
- production of lutein from marine microalgal biomass;
- an efficient process for the production of lutein from marine microalgal biomass;
- an economical process for the production of lutein from marine microalgal biomass; and
- minimal usage of organic solvents as fluid medium for the production of lutein from marine microalgal biomass.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the invention to achieve one or more of the desired objects or results. Variations or modifications to the formulation of this invention, within the scope of the invention, may occur to those skilled in the art upon reviewing the disclosure herein..

The numerical values given for various physical parameters, dimensions and quantities are only approximate values and it is envisaged that the values higher than the numerical value assigned to the physical parameters, dimensions and quantities fall within the scope of the invention unless there is a statement in the specification to the contrary.

While considerable emphasis has been placed herein on the specific features of the preferred embodiment, it will be appreciated that many additional features can be added and that many changes can be made in the preferred embodiment without departing from the principles of the disclosure. These and other changes in the preferred embodiment of the disclosure will be apparent to those skilled in the art from the disclosure herein, whereby it is to be distinctly understood that the foregoing descriptive matter is to be interpreted merely as illustrative of the disclosure and not as a limitation.

## Claims

1. A process for the production of lutein from harvested microalgal biomass, said process comprising the following steps:
i. subjecting said dried harvested microalgal biomass having particle size in the range of 75 microns to 100 microns, to one of supercritical fluid extraction process or ultrasonication extraction process, to obtain a mixture comprising lutein and at least one fluid medium;
ii. saponifying said mixture, using at least one alkali at a temperature in the range of 55 °C to 65 °C and for a time period in the range of 2 hrs to 2.5 hrs to produce a saponified slurry, wherein the ratio of said alkali to said fluid medium is 1:9;
iii. centrifuging said saponified slurry at a speed in the range of 2500 rpm to 3000 rpm, to separate a wet mass comprising lutein; and
iv. concentrating said wet mass at a temperature in the range of 25 °C to 30 °C to obtain lutein.

2. The process for the production of lutein as claimed in claim 1, wherein said supercritical fluid extraction process comprises the following steps:
- introducing the dried harvested microalgal biomass, having particle size in the range of 75 microns to 100 microns, in an air tight vessel;
- passing supercritical carbon dioxide in said vessel;
- adding at least one fluid medium in said vessel, wherein the ratio of the amount of said microalgal biomass to the amount of said at least one fluid medium is in the range of 1:0.3 to 1:0.6;
- increasing the pressure of said vessel to a pressure in the range of 350 bar to 380 bar, and the temperature of said vessel to a temperature in the range of 60 °C to 65 °C for a time period ranging from 0.5 hrs to 4 hrs;
- stirring the contents of said vessel to obtain a slurry stream containing lutein; and
- reducing the pressure of said slurry stream to a pressure in the range of 10 bars to 20 bars to obtain said mixture containing lutein and at least one fluid medium.

3. The process for the production of lutein as claimed in claim 1, wherein said ultrasonication extraction process comprises the following steps:
a. introducing the dried harvested microalgal biomass having particle size in the range of 75 microns to 100 microns, in a vessel;
b. adding at least one fluid medium in the vessel containing said dried microalgal biomass, wherein the ratio of the microalgal biomass to said at least one fluid medium is in the range of 1:10 to 1:40, to obtain a mixture comprising said microalgal biomass and said at least one fluid medium;
c. stirring said mixture for a time period ranging from 2 minutes to 10 minutes to obtain a slurry; and
d. ultrasonicating said slurry at an acoustic intensity of 167 W/cm² and an ultrasonic frequency of 20 kHz, for a time period ranging from 15 minutes to 60 minutes in a chilled bath to obtain said mixture containing lutein and at least one fluid medium.

4. The process as claimed in claim 1, wherein said process further comprises a step of treating said lutein with a salt solution comprising at least one salt selected from the group consisting of NaCl and KCl.

5. The process as claimed in any one of the preceding claims, wherein said microalgal biomass comprises at least one microalgae selected from the group consisting of *Chlorella vulgaris, Chlorella sorokiniana, Chlorella pyrenoidosa, Neochloris aquatica, Dunaliella salina* and *Tetraselmis gracilis.*

6. The process as claimed in claim 1, wherein said alkali is at least one selected from the group consisting of KOH and NaOH.

7. The process as claimed in any one of claims 1 to 3, wherein said at least one fluid medium is selected from the group consisting of ethanol, isoproponal, and a mixture containing ethanol and tetrahydrofuran in the ratio of 2:1.

8. The process as claimed in claim 7, wherein the concentration of said at least one fluid medium is in the range of 98% to 99.8%.

## Patentansprüche

1. Verfahren zur Herstellung von Lutein aus geernteter Mikroalgen-Biomasse, wobei das Verfahren die folgenden Schritte umfasst:
i. Aussetzen der getrockneten geernteten Mikroalgen-Biomasse mit einer Partikelgröße im Bereich von 75 Mikron bis 100 Mikron einem superkritischen Flüssigkeitsextraktionsverfahren oder einem Ultraschall-Extraktionsverfahren, um eine Mischung zu erhalten, die Lutein und mindestens ein flüssiges Medium enthält;
ii. Verseifen der Mischung mittels mindestens einem Alkali bei einer Temperatur im Bereich von 55 °C bis 65 °C und für einen Zeitraum im Bereich von 2 Stunden bis 2,5 Stunden, um eine verseifte Aufschlämmung herzustellen, bei der das Verhältnis des Alkali zum flüssigen Medium 1 : 9 beträgt;
iii. Zentrifugieren der verseiften Aufschlämmung mit einer Drehzahl im Bereich von 2500 U/min bis 3000 U/min, um eine feuchte Masse abzutrennen, die Lutein enthält; und
iv. Verdichten der feuchten Masse bei einer Temperatur im Bereich von 25 °C bis 30 °C, um Lutein zu erhalten.

2. Verfahren zur Herstellung von Lutein nach Anspruch 1, wobei das superkritische Flüssigkeitsextraktionsverfahren die folgenden Schritte umfasst:
- Einleiten der getrockneten geernteten Mikrolagen-Biomasse mit einer Partikelgröße im Bereich von 75 Mikron bis 100 Mikron in einen luftdichten Behälter;
- Durchleiten von superkritischem Kohlendioxid in dem Behälter;
- Zugeben von mindestens einem flüssigem Medium in dem Behälter, wobei das Verhältnis der Menge der Mikroalgen-Biomasse zur Menge des mindestens einen flüssigen Mediums im Bereich von 1 : 0,3 bis 1 : 0,6 liegt;
- Erhöhen des Drucks des Behälters auf einen Druck im Bereich von 350 bar bis 380 bar und der Temperatur des Behälters auf eine Temperatur im Bereich von 60 °C bis 65 °C für einen Zeitraum im Bereich von 0,5 Stunden bis 4 Stunden;
- Rühren des Inhalts des Behälters, um einen aufgeschlämmten Strom zu erhalten, der Lutein enthält; und
- Senken des Drucks des aufgeschlämmten Stroms auf einen Druck im Bereich von 10 bar bis 20 bar, um die Mischung zu erhalten, die Lutein und mindestens ein flüssiges Medium enthält.

3. Verfahren zur Herstellung von Lutein nach Anspruch 1, wobei das Ultraschall-Extraktionsverfahren die folgenden Schritte umfasst:
a. Einleiten der getrockneten geernteten Mikrolagen-Biomasse mit einer Partikelgröße im Bereich von 75 Mikron bis 100 Mikron in einen Behälter;
b. Zugeben von mindestens einem flüssigen Medium in den Behälter, der die getrocknete Mikroalgen-Biomasse enthält, wobei das Verhältnis der Mikroalgen-Biomasse zu dem mindestens einen flüssigen Medium im Bereich von 1 : 10 bis 1 : 40 liegt, um eine Mischung zu erhalten, die die Mikroalgen-Biomasse und das mindestens eine flüssige Medium enthält;
c. Rühren der Mischung für einen Zeitraum im Bereich von 2 Minuten bis 10 Minuten, um eine Aufschlämmung zu erhalten; und
d. Ultraschallbehandlung der Aufschlämmung mit einer Schallstärke von 167 W/cm² und einer Ultraschallfrequenz von 20 kHz für einen Zeitraum im Bereich von 15 Minuten bis 60 Minuten in einem Kühlbad, um die Mischung zu erhalten, die Lutein und mindestens ein flüssiges Medium enthält.

4. Verfahren nach Anspruch 1, wobei das Verfahren ferner einen Schritt der Behandlung des Luteins mit einer Salzlösung umfasst, die mindestens ein Salz enthält, das aus der Gruppe bestehend aus NaCl und KCl gewählt wird.

5. Verfahren nach einem der vorhergehenden Ansprüche, wobei die Mikroalgen-Biomasse mindestens eine Mikroalge umfasst, die aus der Gruppe bestehend aus *Chlorella vulgaris, Chlorella sorokiniana, Chlorella pyrenoidosa, Neochloris aquatica, Dunaliella salina* und *Tetraselmis gracilis* gewählt wird.

6. Verfahren nach Anspruch 1, wobei das Alkali mindestens eins aus der Gruppe bestehend aus KOH und NaOH ist.

7. Verfahren nach einem der Ansprüche 1 bis 3, wobei das mindestens eine flüssige Medium aus der Gruppe bestehend aus Ethanol, Isopropanol sowie einer Mischung, die Ethanol und Tetrahydrofuran im Verhältnis von 2 : 1 enthält, gewählt wird.

8. Verfahren nach Anspruch 7, wobei die Konzentration des mindestens einen flüssigen Mediums im Bereich von 98 % bis 99,8 % liegt.

## Revendications

1. Procédé de production de lutéine à partir de biomasse de microalgues récoltée, ledit procédé comprenant les étapes suivantes :
i. la soumission de ladite biomasse de microalgues séchée et récoltée, ayant une taille de particules comprise entre 75 et 100 microns, à un procédé d'extraction par fluide supercritique ou à un procédé d'extraction par ultrasons, pour obtenir un mélange comprenant de la lutéine et au moins un milieu fluide ;
ii. la saponification dudit mélange, en utilisant au moins un alcali à une température comprise entre 55°C et 65°C et pendant une période comprise entre 2 heures et 2,5 heures pour produire une boue saponifiée, dans laquelle le rapport dudit alcali audit milieu fluide est de 1:9;
iii. la centrifugation de ladite boue saponifiée à une vitesse comprise entre 2 500 et 3 000 tr/min, pour séparer une masse humide comprenant de la lutéine
iv. la concentration de ladite masse humide à une température comprise entre 25 °C et 30 °C pour obtenir de la lutéine.

2. Procédé de production de lutéine selon la revendication 1, dans lequel ledit procédé d'extraction par fluide supercritique comprend les étapes suivantes :
- l'introduction de la biomasse de microalgues séchée et récoltée, dont la taille des particules est comprise entre 75 et 100 microns, dans un récipient hermétique ;
- le passage de dioxyde de carbone supercritique dans ledit récipient ;
- l'ajout d'au moins un milieu fluide dans ledit récipient, dans lequel le rapport de la quantité de ladite biomasse de microalgues à la quantité dudit au moins un milieu fluide est comprise entre 1:0.3 et 1:0.6 ;
- l'augmentation de la pression dudit récipient à une pression comprise entre 350 bar et 380 bar, et la température dudit récipient à une température comprise entre 60°C et 65°C pour une période comprise entre 0,5 heures et 4 heures ;
- l'agitation du contenu dudit récipient pour obtenir un flux de boue contenant de la lutéine; et
- La réduction de la pression dudit flux de boue à une pression comprise entre 10 bars et 20 bars afin d'obtenir ledit mélange contenant de la lutéine et au moins un milieu fluide.

3. Procédé de production de lutéine selon la revendication 1, dans lequel ledit procédé d'extraction par fluide par ultrasons comprend les étapes suivantes :
a. l'introduction de la biomasse de microalgues séchée et récoltée, dont la taille des particules est comprise entre 75 et 100 microns, dans un récipient ;
b. l'ajout d'au moins un milieu fluide dans le récipient contenant ladite biomasse de microalgues séchée, dans lequel le rapport de la biomasse de microalgues audit au moins un milieu fluide est compris entre 1:10 et 1:40, pour obtenir un mélange comprenant ladite biomasse de microalgues et ledit au moins un milieu fluide ;
c. l'agitation dudit mélange pendant une période comprise entre 2 et 10 minutes pour obtenir une boue; et
d. la mise en ultrasons de ladite boue à une intensité acoustique de 167 W/cm2 et à une fréquence ultrasonique de 20 kHz, pendant une durée comprise entre 15 minutes et 60 minutes dans un bain réfrigéré pour obtenir ledit mélange contenant de la lutéine et au moins un milieu fluide.

4. Procédé selon la revendication 1, dans lequel ledit procédé comprend en outre une étape de traitement de ladite lutéine avec une solution salée comprenant au moins un sel choisi dans le groupe constitué de NaCL et KCl.

5. Procédé selon l'une quelconque des revendications précédentes, dans lequel ladite biomasse de microalgues comprend au moins une microalgue choisie dans le groupe constitué de *Chlorella vulgaris, Chlorella sorokiniana, Chlorella pyrenoidosa, Neochloris aquatica, Dunaliella salina* et *Tetraselmis gracilis.*

6. Procédé selon la revendication 1, dans lequel l'alcali est au moins un choisi dans le groupe constitué de KOH et de NaOH.

7. Procédé selon l'une quelconque des revendications 1 à 3, dans lequel ledit au moins un milieu fluide est choisi dans le groupe constitué d'éthanol, d'isoproponal, et un mélange contenant de l'éthanol et du tétrahydrofurane dans un rapport de 2:1.

8. Procédé selon la revendication 7 dans lequel la concentration dudit au moins milieu fluide est comprise entre 98% et 99,8%.
